# EUROPÄISCHE PATENTANMELDUNG

(11) **EP 2 392 454 A1**
(43) Veröffentlichungstag der Anmeldung: **07.12.2011**
(21) Anmeldenummer: 11166664.0
(22) Anmeldetag: 19.05.2011
(51) Int. Cl.: B32B 7/12, C08G 18/77, C09J 175/00, C08K 5/29

(54) **Neuartige flexible Verpackungen, Verfahren zu deren Herstellung und deren Verwendung**

(30) Priorität: 02.06.2010 EP 10164744; 07.02.2011 EP 11153498
(71) Anmelder: Rhein Chemie Rheinau GmbH, 68219 Mannheim (DE)
(72) Erfinder: Cano Sierra, Ana Maria, 69117 Heidelberg (DE); Scheffner, Christian, 69207 Sandhausen (DE); Fruth, Andrea, 65189 Wiesbaden (DE); Ernst, Bruce, 69469 Weinheim (DE)
(74) Vertreter: Siegers, Britta

(57) **Zusammenfassung**

Die Erfindung betrifft neuartige flexible Verpackungen aus mindestens 2 verschiedenen Materialschichten, die mittels Klebeschicht(en) verbunden sind, wobei mindestens eine dieser Klebeschicht(en) ein Carbodiimid oder ein Gemisch aus mehreren Carbodiimiden enthält, ein Verfahren zu deren Herstellung und deren Verwendung.

## Beschreibung

Die Erfindung betrifft neuartige flexible Verpackungen aus mindestens 2 verschiedenen Materialschichten, die mittels Klebeschicht(en) verbunden sind, wobei mindestens eine dieser Klebeschicht(en) ein Carbodiimid oder ein Gemisch aus mehreren Carbodiimide enthält, ein Verfahren zu deren Herstellung und deren Verwendung.

Flexible Verpackungen (Flexible Packaging) erlangen eine immer größere Bedeutung, so z.B. bei flexiblen Kunststoffverbunden, Vakuumbeuteln, coextrudierte Weichfolien etc.
So sind z.B. aus US-A 7414091 Polyurethan-basierte Klebstoffe bekannt oder aus US-A- 5731090 mehrschichtige Verpackungen aus OH-terminiertem Polyester und Isocyanat, die z.B. als Beutel benutzt werden.
Diese Systeme haben den Nachteil, dass sie hydrolyseempfindlich sind, was den Anwendungsbereich stark einschränkt und die Lebensdauer beschränkt.

Es besteht daher ein Bedarf an flexiblen Verpackungen aus mindestens 2 verschiedenen Materialschichten, die eine deutlich erhöhte Stabilität vor allem in Bezug auf die Hydrolyse aufweisen und damit über eine längere Lebensdauer verfügen.

Aufgabe der vorliegenden Erfindung war es daher, neue flexible Verpackungen bereitzustellen, die Nachteile des Standes der Technik nicht aufweisen.

Die dieser Erfindung zugrunde liegenden Aufgabe konnte durch die erfindungsgemäßen neuartigen flexiblen Verpackungen gelöst werden, die aus mindestens 2 verschiedenen Materialschichten bestehen, die mittels Klebeschicht(en) verbunden sind, wobei mindestens eine dieser Klebeschicht(en) ein Carbodiimid oder ein Gemisch aus mehreren Carbodiimiden enthält.

Gegenstand der vorliegenden Erfindung sind daher neuartige flexible Verpackungen aus mindestens 2 verschiedenen Materialschichten, die mittels Klebeschicht(en) verbunden sind, dadurch charakterisiert, dass mindestens eine dieser Klebeschicht(en) ein Carbodiimid oder ein Gemisch von Carbodiimide der Formel (I)

R'-(-N=C=N-R"'-)m-R" (I),

in der
R'" einen aromatischen und/oder araliphatischen Rest bedeutet und bei m ≥1, R'" innerhalb des Moleküls gleich oder verschieden ist und bei verschiedenen Kombinationen jeder der vorgenannten Reste beliebig miteinander kombiniert werden kann,
R'" in dem Fall eines aromatischen oder eines araliphatischen Restes keinen oder in mindestens einer ortho-Stellung zum aromatischen Kohlenstoffatom, das die Carbodiimidgruppe trägt, aliphatische und/oder cycloaliphatische Substituenten mit mindestens 2 Kohlenstoffatomen tragen kann, bevorzugt verzweigte oder cyclische aliphatische Reste mit mind. 3 Kohlenstoffatomen, insbesondere Isopropylgruppen tragen kann, die auch Heteroatome, wie z.B. N, S und/oder O, oder aber Imidazolyl, tragen können,
R' = C₁- C₁₈-Alkyl, C₅-C₁₈-Cycloalkyl-, Aryl, C₇-C₁₈- Aralkyl, -R-NHCOS- R¹,
   -R-COO R¹, -R-O R¹, -R-N(R¹)₂, -R-SR¹, -R-OH, -R-NH₂, -R-NHR¹,
   -R-Epoxy, -R-NCO, -R-NHCONHR¹, -R-NHCONR¹R² oder
   -R-NHCOOR³, wobei
   R = aromatischer, aliphatischer, cycloaliphatischer und/oder araliphatischer Rest,
R"= H, -N=C=N-Aryl, -N=C=N-Alkyl, -N=C=N-Cycloalkyl, -N=C=N-Aralkyl, -NCO, -NHCONHR¹, -NHCONR¹R² , -NHCOOR³, -NHCOS- R¹, -COO R¹, -O R¹, -N (R¹)₂, -SR¹, -OH, -NH₂, -NH R¹,
wobei in R' und R" unabhängig voneinander R¹ und R² gleich oder verschieden sind und einen C₁ - C₂₀-Alkyl-, C₃-C₂₀-Cycloalkyl-, -Aryl, C₇-C₁₈-Aralkylrest, Oligo-/Polyethylen-und/oder -Propylenglykole darstellen und R³ eine der Bedeutungen von R¹ hat oder einen Polyester- oder einen Polyamidrest bedeutet, und m einer ganzen Zahl von 1 bis 5.000 entspricht,
   und/ oder der Formel (II) 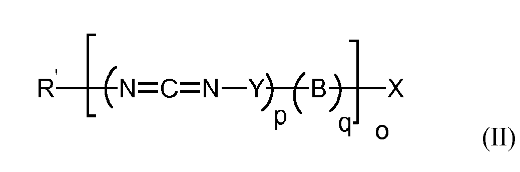 enthält, mit
   Y = C₁-C₁₈-Alkylen-, C₅-C₁₈-Cycloalkylen-, Arylen, C₇-C₁₈-Aralkylen,
   p = eine ganze Zahl von 1 - bis 500, bevorzugt 1 bis 100,
   B = -NH-CO-NH-Z-, -NH-COO-Z-, -NH-COS-Z-,
   q = eine ganze Zahl von 1 bis 500, bevorzugt 1 bis 100,
   o = eine ganze Zahl von 1 bis 500, bevorzugt 1 bis 100,
   X = H, - OH, -SH, -NH₂, -OR¹, -N(R¹)₂, -SR¹, -NHR¹, NR¹R², -OCO-NH- R', NH CO-, -NH-R', -S-CO-NH- R'
   R'= C₁-C₁₈-Alkyl, C₅-C₁₈-Cycloalkyl, Aryl, C₇-C₁₈-Aralcyl,
      - R"'-NH-COS-R¹, -R"'-COOR¹, -R"'-OR¹, -R"'-N(R¹)₂,
      - R"'-SR¹, -R"'-OH, -R'"-NH₂, -R'"-NHR¹, -R"'-Epoxy,
      - R"'-NCO, -R"'-NHCONHR¹, -R"'-NHCONR¹R² oder - R"'-NH-COOR³,
   wobei
R¹ und R² gleich oder verschieden sind und einen C₁-C₂₀ Alkyl, C₃C₂₀-Cycloalkyl-, Aryl, C₇-C₁₈-Arylkylrest, Oligo-/Polyethylen- und/oder Propylenglykole darstellen und R³ eine der Bedeutungen von R¹ hat oder einen Polyester- oder einen Polyamidrest bedeutet, wie z.B. Acetat- oder Butyl- und/oder Benzoeester und
   Z = Y, -Polyester-, -Polyether- und/oder -Polyamid- und R"' einen aromatischen und/oder araliphatischen Rest bedeutet. In Bezug auf die bevorzugten Ausführungsformen von R"' wird auf die obigen Ausführungen verwiesen.

Besonders bevorzugt sind dabei aromatische und sterisch gehinderte Carbodiimide und/oder arylaliphatische sterisch gehinderte und/oder aliphatisch sterisch nicht gehinderte Carbodiimide der vorgenannten Formel (I).

Besonders geeignet sind zudem Carbodiimide der allgemeinen Formel (III), 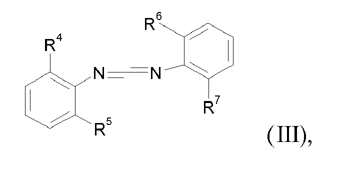 in der R⁴ bis R⁷ unabhängig voneinander linear oder verzweigtes C₁- C₂₀-Alkyl, C₃- bis C₂₀-Cycloalkyl, C₆- C₁₅-Aryl oder einen C₆ -C₁₅-Aralkylrest bedeutet, der gegebenenfalls auch Heteroatome, wie z.B. N, S oder O enthalten kann.

Vorzugsweise entsprechen die Reste R⁴ bis R⁷ C₂- C₂₀-Alkyl oder C₃- C₂₀-Cycloalkylresten.
Ganz besonders bevorzugt entsprechen die Reste R⁴ bis R⁷ C₂ bis C₂₀-Alkylresten.

Ferner können auch polymere aliphatische Carbodiimide verwendet werden, beispielsweise auf Basis von Isophorondiisocyanat oder Dicyclohexylmethan-4,4'-di-isocyanat (H12-MDI = hydriertes MDI).

Ebenso einsetzbar sind biobasierte Carbodiimide, erhältlich aus der Umsetzung von mindestens einem der o.g. Carbodiimide mit mindestens einer freien N-C-O-Funktionalität und einer aus nachwachsenden Rohstoffen isolierten oder daraus hergestellten H-aciden Verbindung und/oder einem Hydroxycarbonsäureester mit 2-24 Kohlenstoffatomen.

Die Materialschichten im Sinne der Erfindung können alle gängigen Polymere enthalten. Vorzugsweise handelt es sich dabei um Polyethylenterephthalat, Polyamide, Polyolefine, wie z.B. Polypropylen und/oder Polyethylen, sowie Aluminiumfolien.

Wichtig bei den erfindungsgemäßen flexiblen Verpackungen ist es, dass die unterschiedlichen Materialschichten durch eine Klebeschicht miteinander verbunden sind. Dabei können 2 - 10 verschiedenen Materialschichten benutzt werden. Ebenfalls möglich ist eine sandwichartige Aufschichtung von 2 oder mehr verschiedenen Materialschichten, z.B. in der Folge:
Materialschicht 1- Klebeschicht - Materialschicht 2 - Klebeschicht - Materialschicht 1- Klebeschicht - Materialschicht 2 oder
Materialschicht 1 - Klebeschicht - Materialschicht 2 - Klebeschicht - Materialschicht 1- Klebeschicht - Materialschicht 3, etc..

In einer weiteren bevorzugten Ausführungsform der Erfindung weist in den Fällen, in den mehrere Klebeschichten existieren, mindestens eine dieser Klebeschichten ein Carbodiimid oder ein Gemisch aus mehreren Carbodiimiden auf. Ganz besonders bevorzugt ist, dass alle Klebeschichten in den flexiblen Verpackungen ein Carbodiimid oder ein Gemisch aus mehreren Carbodiimiden der Formeln (I), (II) und/oder (III) aufweisen.

Bei der Klebeschicht im Sinne der Erfindung können gängigen polymere Klebstoffsysteme, wie lösungsmittel- und/oder wasserbasiert, lösungsmittelfreie und/oder UV-härtende Systeme eingesetzt werden, die nun im Sinne der Erfindung zusätzlich ein Carbodiimid oder ein Gemisch aus mehreren Carbodiimiden der Formeln (I), (II) und/oder (III) enthalten. Die Carbodiimid-freien polymeren Klebstoffsysteme sind handelsüblich, so z.B. bei C.O.I.M erhältlich.

Bevorzugt handelt es sich bei den polymeren Klebstoffsystemen vorzugsweise um polymere Systeme, die ausgewählt werden aus der Gruppe der Polyamide, Copolyamide, Polyolefine, Ethylenvinylacetat-Copolymere, Polyurethane, thermoplastische Polyurethane (TPU), Polyester, Polyacrylate und/oder Vinylpyrrolidon/Vinylacetat-Copolymere, Polyester-Urethane, Acrylate, Polyether und/oder Polyether-Urethane, die zusätzlich ein Carbodiimid oder ein Gemisch aus mehreren Carbodiimide enthalten.

Gegenstand der vorliegenden Erfindung sind daher zudem flexible Verpackungen, bei denen das eingesetzte Carbodiimid erhältlich ist aus der Umsetzung von mindestens einem Carbodiimid der Formel (I) und/oder (II), das über mindestens eine freie N-C-O-Funktionalität verfügt, mit mindestens einer aus nachwachsenden Rohstoffen isolierten oder daraus hergestellten H-aciden Verbindung und/oder einem Hydroxycarbonsäureester mit 2-24-Kohlenstoffatomen.

Bei den Hydroxycarbonsäureestern im Sinne der Erfindung handelt es sich vorzugsweise um Hydroxycarbonsäureester mit 2-24-Kohlenstoffatomen, wie z.B. Polymilchsäure und/oder Polyhydroxybutyrate der Formel (IV) 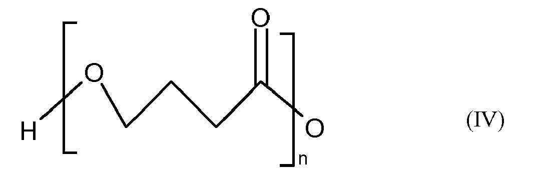 mit n = 2 -20.

Bei den aus nachwachsenden Rohstoffen isolierten H-aciden Verbindungen handelt es sich vorzugsweise um natürliche Polyole, wie z.B. Rizinusöl, Stärke und/oder Zucker.
Bei den vorgenannten Verbindung handelt es sich um handelsübliche Substanzen.

Bei den aus nachwachsenden Rohstoffen hergestellten H-aciden Verbindungen handelt es sich vorzugsweise um Glycerin, Polyole aus Pflanzenölen, wie z.B. Rapsöl, Sojaöl und/oder aus ungesättigten Fettsäuren, wie z.B. Ölsäure.

Bei den vorgenannten Verbindung handelt es sich um handelsübliche Substanzen, die z.B. bei der Firma Cargill und/oder Urethane Soy Systems erhältlich sind.

Die Umwandlung aus Pflanzenölen bzw. aus ungesättigten Fettsäuren kann nach den dem Fachmann geläufigen Verfahren, wie z.B. durch Ozonisierung mit nachfolgender Glykolyse, Epoxidierung mit anschließender Ringöffnung durch z.B. Alkohole oder Hydroformylierung und anschließende Reduktion mit Wasserstoff erfolgen.

In Bezug auf die biobasierten Carbodiimide und deren Herstellung wird auf die Anmeldungen DE-A 102009001130.7 und EP 10163621.5 verwiesen, deren Inhalt hiermit in diese Anmeldung aufgenommen wird.

Unter dem Begriff biobasierte Carbodiimide werden Carbodiimde verstanden, die einen Anteil von mehr als 7% eines biobasierenden Rohstoffes aufweisen.

Bei den vorgenannten Carbodiimiden handelt es sich um käuflich erhältliche Verbindungen, die z.B. bei der Rhein Chemie Rheinau GmbH unter den Handelsnamen Stabaxol® I (N-C-N-Gehalt: 10 %), Stabaxol® P (N-C-N-Gehalt: 12,5-13,5%) und Stabaxol® P 200 (N-C-N-Gehalt: 6-7,5 %) käuflich erhältlich sind.

Ebenso möglich ist auch die Herstellung der Carbodiimide nach den beispielsweise in
US 2,941,956 beschrieben Verfahren oder durch die Kondensation von Diisocyanaten unter Abspaltung von Kohlendioxid bei erhöhten Temperaturen, z.B. bei 40 °C bis 200 °C, in Gegenwart von Katalysatoren. Geeignete Verfahren werden in DE-A-11 30 594 und in der
FR 1 180 370 beschrieben. Als Katalysatoren haben sich z.B. starke Basen oder Phosphorverbindungen bewährt. Vorzugsweise werden Phospholenoxide, Phospholidine oder Phospholinoxide sowie die entsprechenden Sulfide verwendet. Ferner können als Katalysatoren tertiäre Amine, basisch reagierende Metallverbindungen, Carbonsäuremetallsalze und nicht basische Organometallverbindungen verwendet werden.

Zur Herstellung der eingesetzten Carbodiimide und/oder Polycarbodiimide eignen sich alle Isocyanate, wobei im Rahmen der vorliegenden Erfindung bevorzugt Carbodiimide und/oder Polycarbodiimide verwendet werden, die auf durch C₁- bis C₄-Alkyl substituierten aromatischen Isocyanaten aufbauen, wie z.B. 2,6-Diisopropylphenylisocyanat, 2,4,6-Triisopropylphenyl-1,3-diisocyanat, 2,4,6-Triethylphenyl-1,3-diisocyanat, 2,4,6-Trimethyl-phenyl-1,3-diisocyanat, 2,4'-Diisocyanatodiphenylmethan, 3,3',5,5'-Tetraisopropyl-4,4'-diisocyanatodiphenylmethan, 3,3',5,5'-Tetraethyl-4,4'-diisocyanatodiphenylmethan, Tetramethylxyloldiisocyanat, 1,5-Naphthalindiisocyanat, 4,4'-Diphenylmethandiisocyanat, 4,4'-Diphenyldimethyl-methan-diisocyanat, 1,3-Phenylendiisocyanat, 1,4-Phenylendiisocyanat, 2,4-Tolylendiisocyanat, 2,6-Tolylen-diisocyanat, ein Gemisch aus 2,4-Tolylendiisocyanat und 2,6-Tolylendiisocyanat, Hexamethylendiisocyanat, Cyclohexan-1,4-diisocyanat, Xylylendi-isocyanat, Isophoron-diisocyanat, Dicyclohexyhnethan-4,4'-di-isocyanat, Methylcyclohexandi-isocyanat, Tetramethylxylylendiisocyanat, 2,6-Diisopropylphenylenisocyanat und 1,3,5-Triisopropylbenzol-2,4-diisocyanat oder deren Gemische, oder auf substituierten Aralkylen, wie 1,3-Bis-(1-methyl-1-isocyanato-ethyl)-benzol, basieren. Besonders bevorzugt ist es, wenn die Carbodiimide und/oder Polycarbodiimide auf 2,4,6-Triisopropylphenyl-1,3-diisocyanat und/oder 2,6-Diisopropyl-phenylenisocyanat und/oder Tetramethylxylylendiisocyanat basieren.

Polycarbodiimide können außerdem, wenn sie aus Isocyanaten hergestellt worden sind, noch reaktionsfähige NCO-Gruppen und komplex gebundene monomere Isocyanate enthalten.

In einer weiteren Ausfuhrungsform der vorliegenden Erfindung ist es auch möglich, eine Mischung verschiedener Carbodiimide einzusetzen.

Vorzugsweise beträgt die Menge an Carbodiimid 0,01 bis 50 Gew.%, bezogen auf die Klebeschicht. Die polymeren Klebstoffsysteme können neben dem Carbodiimid auch weitere Additive, wie z.B. Antioxidantien, Flammschutzmittel, Lösungsmittel, wie z.B. Ethylacetat oder Wasser, enthalten.

Die zur Herstellung der erfindungsgemäßen flexiblen Verpackung eingesetzte Klebeschicht wird dabei vorzugsweise wie folgt hergestellt:
Ein oder mehrere polymere Systeme, die ausgewählt werden aus der Gruppe der Polyamide, Copolyamide, Polyolefine, Ethylenvinylacetat-Copolymere, Polyurethane, thermoplastische Polyurethane (TPU), Polyester, Polyacrylate und/oder Vinylpyrrolidon/Vinylacetat-Copolymere, Polyester-Urethane, Acrylate, Polyether und/oder Polyether-Urethane, werden mit mindestens einem Carbodiimid oder einem Gemisch aus mehreren Carbodiimiden und gegebenenfalls weiteren Additiven versetzt.

Gegenstand der vorliegenden Erfindung ist zudem ein Verfahren zur Herstellung der erfindungsgemäßen flexiblen Verpackungen, wonach auf die Oberfläche mindestens einer Materialschicht die Klebstoffschicht aus polymeren Systemen aufgerakelt wird, die ausgewählt werden aus der Gruppe der Polyamide, Copolyamide, Polyolefine, Ethylenvinylacetat-Copolymere, Polyurethane, thermoplastische Polyurethane (TPU), Polyester, Polyacrylate und/oder Vinylpyrrolidon/Vinylacetat-Copolymere, Polyester-Urethane, Acrylate, Polyether und/oder Polyether- Urethane, und die zusätzlich mindestens ein Carbodiimid oder ein Gemisch aus mehreren Carbodiimiden der Formel (I)

R'-(-N=C=N-R"'-)m-R" (I),

in der
R"' einen aromatischen und/oder araliphatischen Rest bedeutet und bei m ≥1, R"' innerhalb des Moleküls gleich oder verschieden ist und bei verschiedenen Kombinationen jeder der vorgenannten Reste beliebig miteinander kombiniert werden kann,
R'" in dem Fall eines aromatischen oder eines araliphatischen Restes keinen oder in mindestens einer ortho-Stellung zum aromatischen Kohlenstoffatom, das die Carbodiimidgruppe trägt, aliphatische und/oder cycloaliphatische Substituenten mit mindestens 2 Kohlenstoffatomen tragen kann, die auch Heteroatome tragen können,
R' = C₁- C₁₈-Alkyl, C₅-C₁₈-Cycloalkyl-, Aryl, C₇-C₁₈- Aralkyl, -R-NHCOS- R¹,
   -R-COO R¹, -R-O R¹, -R-N(R¹)₂, -R-SR¹, -R-OH, -R-NH₂, -R-NHR¹,
   -R-Epoxy, -R-NCO, -R-NHCONHR¹, -R-NHCONR¹R² oder
   -R-NHCOOR³ , mit R = aromatischer, aliphatischer, cycloaliphatischer und/oder araliphatischer Rest,
R"= H, -N=C=N-Aryl, -N=C=N-Alkyl, -N=C=N-Cycloalkyl, -N=C=N-Aralkyl, -NCO, -NHCONHR¹, -NHCONR¹R², -NHCOOR³, -NHCOS- R¹, -COO R¹, -O R¹, -N (R¹)₂, -SR¹, -OH, -NH₂, -NH R¹,
wobei in R' und R" unabhängig voneinander R¹ und R² gleich oder verschieden sind und einen C₁-C₂₀-Alkyl-, C₃-C₂₀-Cycloalkyl-, -Aryl, C₇-C₁₈-Aralkylrest, Oligo-/Polyethylen- und/oder -Propylenglykole darstellen und R³ eine der Bedeutungen von R¹ hat oder einen Polyester- oder einen Polyamidrest bedeutet, und m einer ganzen Zahl von 1 bis 5.000 entspricht, und/oder der Formel (II) 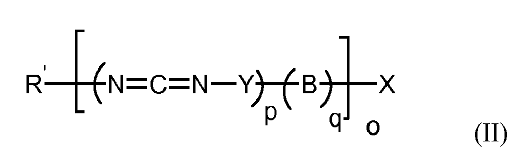 enthalten, mit
   Y = C₁-C₁₈-Alkylen-, C₅-C₁₈-Cycloalkylen-, Arylen, C₇-C₁₈-Aralkylen,
   p = eine ganze Zahl von 1 -bis 500, bevorzugt 1 bis 100,
   B = -NH-CO-NH-Z-, -NH-COO-Z-, -NH-COS-Z-,
   q = eine ganze Zahl von 1 bis 500, bevorzugt 1 bis 100,
   o = eine ganze Zahl von 1 bis 500, bevorzugt 1 bis 100,
   X = H, - OH, -SH, -NH₂, -OR¹, -N(R¹)₂, -SR¹, -NHR¹, NR¹R² -OCO-NH- R', NH CO-, -NH-R', -S-CO-NH- R'
   R'= C₁-C₁₈-Alkyl, C₅-C₁₈-Cycloalkyl, Aryl, C₇-C₁₈-Aralcyl
      - R"'-NH-COS-R¹, -R"'-COOR¹, -R"'-OR¹, -R"'-N(R¹)₂,
      - R"'-SR¹, -R"'-OH, -R'"-NH₂, -R"'-NHR1, -R"'-Epoxy,
      - R"'-NCO, -R"'-NHCONHR¹, -R"'-NHCONR¹R² oder
   - R"'-NH-COOR³,
   wobei
R¹ und R² gleich oder verschieden sind und einen C₁-C₂₀ Alkyl, C₃-C₂₀-Cycloalkyl-, Aryl, C₇-C₁₈-Arylkylrest, Oligo-/Polyethylen- und/oder Propylenglykole darstellen und R³ eine der Bedeutungen von R¹ hat oder einen Polyester- oder einen Polyamidrest bedeutet, wie z.B. Acetat- oder Butyl- und/oder Benzoeester und R'" einen aromatischen und/oder araliphatischen Rest bedeutet,
   Z = Y, -Polyester-, -Polyether- und/oder -Polyamid-,
und die gegebenenfalls weitere Additive enthalten,
und die weitere(n) Materialschichten auf diese Klebstoffschicht aufgelegt und die Klebstoftschicht(en) ausgehärtet wird/werden.

In Bezug auf die bevorzugten Ausführungsformen von R'" wird auf die obigen Ausführungen verwiesen.

Bei den Carbodiimiden handelt es sich dabei vorzugsweise um mindestens eine der bereits weiter oben beschriebenen Verbindungen der Formeln (I), (II) und/oder (III) oder um biobasierte Carbodiimide, die erhältlich sind aus der Umsetzung von mindestens einem Carbodiimid der Formel (I) und/oder (II), das über mindestens eine freie N-C-O-Funktionalität verfügt, mit mindestens einer aus nachwachsenden Rohstoffen isolierten oder daraus hergestellten H-aciden Verbindung und/oder einem Hydroxycarbonsäureester mit 2-24-Kohlenstoffatomen.

In diesem Zusammenhang wird auf die zuvor genannten Definitionen der Verbindungen der Formeln (I), (II) und/oder (III) und (IV) die zuvor genannten Definitionen der biobasierten Verbindungen verwiesen.

Um Oberflächen für Verklebungen vorzubereiten sind vielfältige Möglichkeiten der Vorbehandlung bekannt. Generell sollten die Materialschicht erst fettfrei sein, bevor mit anderen Vorbehandlungsmethoden weiter gereinigt wird. Um dies sicherzustellen sind Reinigungsschritte zum Beispiel mit Lösungsmitteln oder eine Vortrocknung im Ofen möglich. Für Hochleistungsverklebungen gibt es weitere Methoden wie beispielsweise Beflammen, Niederdruckplasma- oder Normaldruckplasma-Behandlung und die Koronaentladungstechnik. Weiterhin ist eine Vorbehandlung mit Haftvermittlern (Primer, Haftprimer) möglich.

In einer weiteren Ausführungsform der Erfindung erfolgt eine Vorbehandlung der Materialschicht vor dem Aufrakeln der Klebstoffschicht.

In einer bevorzugten Ausführungsform der Erfindung erfolgt das Aushärten bei Temperaturen zwischen 20 und 100°C gegebenenfalls bei Drücken zwischen 1 und 50 bar.

Vorzugsweise beträgt die Dicke der aufgerakelten Klebstoffschicht 10 bis 250 µm.

Das Aufrakeln kann dabei unter Einsatz von Maschinen erfolgen, wie beispielsweise mit herkömmlichen Laminiermaschinen. Besonders geeignet ist der Auftrag der Klebstoffschicht im flüssigen Zustand auf die zu verklebende Materialschicht, vorzugsweise eine Folie, wie beispielsweise einer Folie aus Kunststoff oder Metall.

Typische Verarbeitungstemperaturen sind beispielsweise etwa 25°C bis 130°C, vorzugsweise bis 75°C bei der Herstellung flexibler Verpackungsfolien (flexible packaging).

Das beschriebene Verfahren kann mehrfach wiederholt werden, so dass Folienverbunde hergestellt werden können, die aus mehr als zwei verklebten Materialschichten bestehen.

Gegenstand der vorliegenden Erfindung ist zudem die Verwendung von polymeren Systemen, die ausgewählt sind aus der Gruppe der Polyamide, Copolyamide, Polyolefine, Ethylenvinylacetat-Copolymere, Polyurethane, thermoplastische Polyurethane (TPU), Polyester, Polyacrylate und/oder Vinylpyrrolidon/Vinylacetat-Copolymere, Polyester-Urethane, Acrylate, Polyether und/oder Polyether-Urethane, die zusätzlich mindestens ein Carbodiimid oder ein Gemisch aus mehreren Carbodiimiden der Formeln (I), (II) und/oder (III) und gegebenenfalls weitere Additive enthalten, zum Verkleben von mindesten 2 unterschiedlichen Materialschichten zur Herstellung von flexible Verpackungen.
Ebenfalls eingesetzt werden können dabei auch Verbindungen der Formel (I) und/oder (II), die erhältlich sind aus der Umsetzung von mindestens einem Carbodiimid der Formel (I) und/oder (II), das über mindestens eine freie N-C-O-Funktionalität verfügt, mit mindestens einer aus nachwachsenden Rohstoffen isolierten oder daraus hergestellten H-aciden Verbindung und/oder einem Hydroxycarbonsäureester mit 2-24-Kohlenstoffatomen.

In Bezug auf die Charakterisierung der polymeren Systeme und der Carbodiimide der Formeln (I), (II) und/oder (III) sowie der Hydroxycarbonsäureester wird auf die obigen Ausführungen verwiesen.

Gegenstand der vorliegenden Erfindung ist daher zudem die Verwendung der erfindungsgemäßen flexiblen Verpackung für den Einsatz im Lebensmittelsmittelbereich, für medizinische Anwendungen, wie z.B. Infusionslösungen, Verpackung von Spritzen etc. und zur Verpackung industrieller Produkte.

Die nachfolgenden Beispiele dienen der Erläuterung der Erfindung, ohne dabei limitierend zu wirken.

### Ausführungsbeispiele

Aus den nachstehend genannten Ausgangsstoffen wurden die folgende Proben VV und 1- 4 hergestellt:
Desmocoll® 140, ein im wesentlichen lineares Hydroxy-Polyurethan der Firma BayerMaterial Science AG.
Baycoll® AS 2060, ein schwach verzweigtes Polyesterpolyol der Firma Bayer MaterialScience AG.
Desmodur® RFE, eine Lösung von Tris(p-isocyanatophenyl)thiophosphat in Ethylacetat.
Carbodiimid A, ein oligomeres, aromatisches Carbodiimid mit einem N=C=N-Gehalt von mindestens 12,5%, erhältlich bei der Rhein Chemie Rheinau GmbH unter dem Namen Stabaxol®P,
Carbodiimid B, ein monomeres Carbodiimid: Bis-(2,6-diisopropylphenyl)-Carbodiimid mit einem N=C=N-Gehalt von mindestens 10%, erhältlich bei der Rhein Chemie Rheinau GmbH unter dem Namen Stabaxol® I,
Carbodiimid C: Bis-(2,6-diisopropylphenyl)-Carbodiimid (monomer, low fogging, niedriger VOC-(volatile organic compounds)-Anteil durch reduzierten Monomergehalt) mit einem N=C=N-Gehalt von mindestens 10%, erhältlich bei der Rhein Chemie Rheinau GmbH unter dem Namen Stabaxol® I LF,
Carbodiimid D: ein polymeres Carbodiimid mit einem N=C=N-Gehalt: 6-7,5% erhältlich bei der Rhein Chemie Rheinau GmbH unter dem Namen Stabaxol® P 200.
Die Carbodiimide A- D wurden in den Mengenverhältnissen eingesetzt, dass in den Beispielen 1 - 4 jeweils derselbe -N=C=N-Gehalt vorliegt.
Die in der nachstehenden Tabelle 1 angegebenen Anteile sind in phr.

**Tabelle 1: Einsatzmengen zur Herstellung der Klebestoffe**

| Komponente | VV | 1 (erfindungsgemäß) | 2 (erfindungsgemäß) | 3 (erfindungsgemäß) | 4 (erfindungsgemäß) |
|---|---|---|---|---|---|
| Desmocoll® 140 | 14 | 14 | 14 | 14 | 14 |
| Baycoll® AS 2060 | 7 | 7 | 7 | 7 | 7 |
| Desmodur® RFE | 4 | 4 | 4 | 4 | 4 |
| Ethylacetat | 75 | 75 | 75 | 75 | 75 |
| Carbodiimid A | x | 0,25 | x | x | x |
| Carbodiimid B | x | x | 0,32 | x | x |
| Carbodiimid C | x | x | x | 0,32 | x |
| Carbodiimid D | x | x | x | x | 0,48 |

Dabei wurden die Klebstoffproben wie folgt hergestellt:
Variante 1: 14 phr Desmocoll® 140 wurde bei 85°C in 75 phr wasserfreiem Ethylacetat gelöst. Beim Abkühlen der Lösung auf Raumtemperatur wurden die übrigen, in der Tabelle 1 genannten Komponenten zugegeben.
Variante 2: 14 phr Desmocoll® 140 wurde bei 85°C in 75 phr wasserfreiem Ethylacetat gelöst. Beim Abkühlen der Lösung auf Raumtemperatur wurden die übrigen, in der Tabelle 1 genannten Komponenten bis auf Desmodur® RFE zugegeben. Danach wurde über einen Zeitraum von 5 Stunden auf 80°C erhitzt und anschließend wurde Desmodur® RFE zugegeben.

Herstellung von Probekörpern (Peelproben) unter Einsatz der Proben VV und 1-4, hergestellt nach den Varianten 1 bzw. 2:
Zur Herstellung der Peelproben wurde eine handelsübliche PET-Folie der Firma Goodfellow GmbH der Dicke 23 µm mit einer Aluminiumfolie der Firma Goodfellow GmbH der Dicke 100 µm eingesetzt. Die Folien wurden nicht vorbehandelt.

Die o.g. Klebstoffproben VV und 1- 4, hergestellt nach den Varianten 1 bzw. 2, wurden jeweils mit einer Nassschichtdicke von 50 µm auf die Aluminiumfolien aufgerakelt Nach einer 10 minütigen Lagerung wird die PET-Folie auf die jeweilige Klebstoffschicht aufgelegt und bei 40°C für eine Stunde bei einem Anpressdruck von 3 Kilogramm ausgehärtet.

Aus diesen PET-Aluminium-Folienverbunden werden Prüfkörper der Größe 20 x 30 cm hergestellt.

Die hervorragende Langzeitstabilität der Klebeschicht der aus mindestens 2 Schichten bestehenden Folien (PET-Aluminium-Folien) wird anhand des nachstehend beschriebenen Rollenschälversuch vor und nach der Alterung demonstriert.

Beim Rollenschälversuch wird der Schälwiderstand eines Klebstoffes in N/cm oder N/mm bestimmt. In diesem Test wurde das Ende der PET-Aluminium-Folienverbunde um jeweils 90° umgebogen, so dass die Probe anschließend wie ein T-Stück aussah. Die freien Enden wurden in einer Universalprüfmaschine eingespannt und auseinander gezogen. Die Werte für über die Varianten 1 bzw. 2 hergestellten PET-Aluminium-Folienverbunde sind in den nachfolgenden Tabellen 2-4 aufgeführt

**Tabelle 2 nach Variante 1 (ungetempert):**

| Zeit [h] | VV [N/mm] | 1 [N/mm] | 2[N/mm] | 3 [N/mm] | 4 [N/mm] |
|---|---|---|---|---|---|
| 0 | 0,01 | 0,04 | 0,04 | 0,03 | 0,03 |
| 168 | 0,08 | 0,11 | 0,15 | 0,14 | 0,15 |
| 500 | 0,02 | 0,15 | 0,14 | 0,14 | 0,12 |
| 1000 | 0,01 | 0,17 | 0,22 | 0,02 | 0,02 |

**Tabelle 3 nach Variante 1 (getempert nach 60h/45°C)**

| Zeit [h] | VV [N/mm] | 2 [N/mm] | 3 [N/mm] | 4 [N/mm] |
|---|---|---|---|---|
| 0 | 0,08 | 0,10 | 0,07 | 0,03 |
| 168 | 0,16 | 0,15 | 0,15 | 0,09 |
| 500 | 0,17 | 0,18 | 0,18 | 0,10 |
| 1000 | 0,02 | 0,27 | 0,09 | 0,11 |

**Tabelle 4 nach Variante 2 (ungetempert):**

| Zeit | VV [N/mm] | 1 [N/mm] | 2 [N/mm] | 3 [N/mm] | 4 [N/mm] |
|---|---|---|---|---|---|
| 0 | 0,01 | 0,03 | 0,03 | 0,03 | 0,02 |
| 168 | 0,08 | 0,08 | 0,11 | 0,14 | 0,12 |
| 500 | 0,02 | 0,10 | 0,15 | 0,16 | 0,11 |
| 1000 | 0,01 | 0,11 | 0,10 | 0,10 | 0,11 |
| 2040 | 0 | 0,01 | 0,02 | 0,02 | 0,06 |

Alle erfindungsgemäßen Proben zeigten positive Ergebnisse im Rollenschälversuch über einen sehr langen Zeitraum. Dies bedeutet, dass die erfindungsgemäßen Proben gegenüber dem Stand der Technik (VV) eine deutlich erhöhte Stabilität aufweisen und damit über eine längere Lebensdauer verfügen. Dies ist auch ein Beleg für die erhöhte Hydrolysestabilität.

## Patentansprüche

1. Flexible Verpackungen aus mindestens 2 verschiedenen Materialschichten, die mittels Klebeschicht(en) verbunden sind, **dadurch gekennzeichnet, dass** mindestens eine dieser Klebeschicht(en) ein Carbodiimid oder ein Gemisch aus mehreren Carbodiimiden der Formel (I)
R'-(-N=C=N-R"'-)ₘ R" (I),
in der
R'" einen aromatischen und/oder araliphatischen Rest bedeutet und bei m ≥1, R'" innerhalb des Moleküls gleich oder verschieden ist und bei verschiedenen Kombinationen jeder der vorgenannten Reste beliebig miteinander kombiniert werden kann,
R'" in dem Fall eines aromatischen oder eines araliphatischen Restes keinen oder in mindestens einer ortho-Stellung zum aromatischen Kohlenstoffatom, das die Carbodiimidgruppe trägt, aliphatische und/oder cycloaliphatische Substituenten mit mindestens 2 Kohlenstoffatomen tragen kann, die auch Heteroatome tragen können,
R' = C₁- C₁₈-Alkyl, C₅-C₁₈-Cycloalkyl-, Aryl, C₇-C₁₈- Aralkyl, -R-NHCOS- R¹,
-R-COO R¹, -R-O R¹, -R-N(R¹)₂, -R-SR¹, -R-OH, -R-NH₂, -R-NHR¹,
-R-Epoxy, -R-NCO, -R-NHCONHR¹, -R-NHCONR¹R² oder
-R-NHCOOR³ , wobei R = aromatischer, aliphatischer, cycloaliphatischer und/oder araliphatischer Rest,
R"= H, -N=C=N-Aryl, -N=C=N-Alkyl, -N=C=N-Cycloalkyl, -N=C=N-Aralkyl, -NCO, -NHCONHR , -NHCONR¹R² , -NHCOOR³, -NHCOS- R¹, -COO R¹, -O R¹, -N (R¹)₂, -SR¹, -OH, -NH₂, -NH R¹,
wobei in R' und R" unabhängig voneinander R¹ und R² gleich oder verschieden sind und einen C₁ - C₂₀-Alkyl-, C₃-C₂₀-Cycloalkyl-, -Aryl, C₇-C₁₈-Aralkylrest, Oligo-/Polyethylen-und/oder -Propylenglykole darstellen und R³ eine der Bedeutungen von R¹ hat oder einen Polyester- oder einen Polyamidrest bedeutet, undm einer ganzen Zahl von 1 bis 5.000 entspricht,
und/oder mindestens ein Carbodiimid der Formel (II) 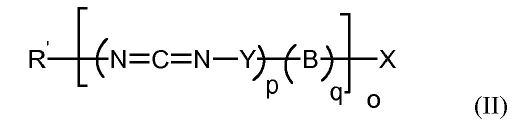 enthält, mit
Y = C₁-C₁₈-Alkylen-, C₅-C₁₈-Cycloalkylen-, Arylen, C₇-C₁₈-Aralkylen,
p = eine ganze Zahl von 1 -bis 500, bevorzugt 1 bis 100,
B = -NH-CO-NH-Z-, -NH-COO-Z-, -NH-COS-Z-,
q = eine ganze Zahl von 1 bis 500, bevorzugt 1 bis 100,
o = eine ganze Zahl von 1 bis 500, bevorzugt 1 bis 100,
X = H, - OH, -SH, -NH₂, -OR¹, -N(R¹)₂, -SR¹, -NHR¹, NR¹R², -OCO-NH- R', NH CO-, -NH-R', -S-CO-NH- R'
R'= C₁-C₁₈-Alkyl, C₅-C₁₈-Cycloalkyl, Aryl, C₇-C₁₈-Aralcyl
- R"'-NH-COS-R¹, -R"'-COOR¹, -R"'-OR¹, -R"'-N(R¹)₂,
- R"'-SR¹, -R"'-OH, -R"'-NH₂, -R"'-NHR1, -R"'-Epoxy,
- R"'-NCO, -R"'-NHCONHR¹, -R"'-NHCONR¹R² oder
- R"'-NH-COOR³,
wobei
R¹ und R² gleich oder verschieden sind und einen C₁-C₂₀ Alkyl, C₃-C₂₀-Cycloalkyl-, Aryl, C₇-C₁₈-Arylkylrest, Oligo-/Polyethylen- und/oder Propylenglykole darstellen und R³ eine der Bedeutungen von R¹ hat oder einen Polyester- oder einen Polyamidrest bedeutet, R"' einen aromatischen und/oder araliphatischen Rest bedeutet und
Z = Y, -Polyester-, -Polyether- und/oder -Polyamid-,
bedeutet.

2. Flexible Verpackungen nach Anspruch 1, **dadurch gekennzeichnet, dass** es sich bei den Materialschichten um Polyethylenterephthalat, Polyolefine, Polyamide und/oder Aluminiumfolien handelt.

3. Flexible Verpackungen nach Anspruch 1 oder 2, **dadurch gekennzeichnet, dass** es sich bei der Klebeschicht um polymere Systeme handelt, die ausgewählt werden aus der Gruppe der Polyamide, Copolyamide, Polyolefine, Ethylenvinylacetat-Copolymere, Polyurethane, thermoplastische Polyurethane, Polyester, Polyacrylate und/oder Vinylpyrrolidon/VinylacetatCopolymere, Polyester-Urethane, Acrylate, Polyether und/oder Polyether-Urethane.

4. Flexible Verpackungen nach einem oder mehreren der Ansprüche 1 bis 3, **dadurch gekennzeichnet, dass** die Klebeschicht lösungsmittel- und/oder wasserbasiert, lösungsmittelfrei und/oder UV-härtend ist.

5. Flexible Verpackungen nach einem oder mehreren der Ansprüche 1 bis 4, **dadurch gekennzeichnet, dass** es sich bei dem Carbodiimid um eine Verbindung der allgemeinen Formel (III) handelt, 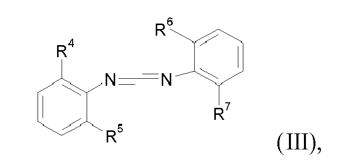 in der R⁴ bis R⁷ unabhängig voneinander linear oder verzweigtes C₁- bis C₂₀-Alkyl, C₃- bis C₂₀-Cycloalkyl, C₆- C₁₅-Aryl oder ein C₆ - C₁₅-Aralkylrest bedeutet, der gegebenenfalls auch Heteroatome enthalten kann.

6. Flexible Verpackungen nach einem oder mehreren der Ansprüche 1 bis 5, **dadurch gekennzeichnet, dass** das eingesetzte Carbodiimid erhältlich ist aus der Umsetzung von mindestens einem Carbodiimid der Formel (I) oder(II)), das über mindestens eine freie N-C-O-Funktionalität verfügt, mit mindestens einer aus nachwachsenden Rohstoffen isolierten oder daraus hergestellten H-aciden Verbindung und/oder einem Hydroxycarbonsäureester mit 2-24-Kohlenstoffatomen.

7. Verfahren zur Herstellung der flexiblen Verpackungen nach einem oder mehreren der Ansprüche 1 bis 6, **dadurch gekennzeichnet, dass** auf die Oberfläche mindestens einer Materialschicht die Klebstoffschicht aus polymerem Systemen aufgerakelt wird, die ausgewählt ist aus der Gruppe der Polyamide, Copolyamide, Polyolefine, Ethylenvinylacetat-Copolymere, Polyurethane, thermoplastische Polyurethane, Polyester, Polyacrylate und/oder Vinylpyrrolidon/Vinylacetat-Copolymere, Polyester-Urethane, Acrylate, Polyether und/oder Polyether- Urethane, und die zusätzlich ein Carbodiimid oder ein Gemisch aus mehreren Carbodiimiden
der Formel (I)
R'-(-N=C=N-R"'-)ₘ R" (I),
in der
R'" einen aromatischen und/oder araliphatischen Rest bedeutet und bei m ≥1, R'" innerhalb des Moleküls gleich oder verschieden ist und bei verschiedenen Kombinationen jeder der vorgenannten Reste beliebig miteinander kombiniert werden kann,
R'" in dem Fall eines aromatischen oder eines araliphatischen Restes keinen oder in mindestens einer ortho-Stellung zum aromatischen Kohlenstoffatom, das die Carbodiimidgruppe trägt, aliphatische und/oder cycloaliphatische Substituenten mit mindestens 2 Kohlenstoffatomen tragen kann, die auch Heteroatome tragen können,
R' = C₁- C₁₈-Alkyl, C₅-C₁₈-Cycloalkyl-, Aryl, C₇-C₁₈- Aralkyl, -R-NHCOS- R¹,
-R-COO R¹, -R-O R¹, -R-N(R¹)₂, -R-SR¹, -R-OH, -R-NH₂, -R-NHR¹,
-R-Epoxy, -R-NCO, -R-NHCONHR¹, -R-NHCONR¹R² oder
-R-NHCOOR³ , wobei R = aromatische, aliphatisch, cycloaliphatisch und/oder araliphatischen Rest,
R"= H, -N=C=N-Aryl, -N=C=N-Alkyl, -N=C=N-Cycloalkyl, -N=C=N-Aralkyl, -NCO, -NHCONHR¹, -NHCONR¹R², -NHCOOR³, -NHCOS- R¹, -COO R¹, -O R¹, -N (R¹)₂, -SR¹, -OH, -NH₂, -NH R¹,
wobei in R' und R" unabhängig voneinander R¹ und R² gleich oder verschieden sind und einen C₁ - C₂₀-Alkyl-, C₃-C₂₀-Cycloalkyl-, -Aryl, C₇-C₁₈-Aralkylrest, Oligo-/Polyethylen-und/oder -Propylenglykole darstellen und R³ eine der Bedeutungen von R¹ hat oder einen Polyester- oder einen Polyamidrest bedeutet, undm einer ganzen Zahl von 1 bis 5.000 entspricht,
und/oder der Formel (II) 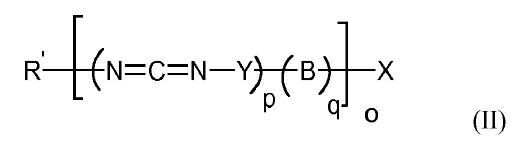 enthält, mit
Y = C₁-C₁₈-Alkylen-, C₅-C₁₈-Cycloalkylen-, Arylen, C₇-C₁₈-Aralkylen,
p = eine ganze Zahl von 1 -bis 500, bevorzugt 1 bis 100
B = -NH-CO-NH-Z-, -NH-COO-Z-, -NH-COS-Z-,
q = eine ganze Zahl von 1 bis 500, bevorzugt 1 bis 100,
o = eine ganze Zahl von 1 bis 500, bevorzugt 1 bis 100,
X = H, - OH, -SH, -NH₂, -OR¹ , -N(R¹)₂ -SR¹, -NHR¹, NR¹R² -OCO-NH- R', NH CO-, -NH-R', -S-CO-NH- R'
R'= C₁-C₁₈-Alkyl, C₅-C₁₈-Cycloalkyl, Aryl, C₇-C₁₈-Aralkyl
- R"'-NH-COS-R¹, -R"'-COOR¹, -R"'-OR¹, -R"'-N(R¹)₂,
- R"'-SR¹, -R"'-OH, -R"'-NH₂, -R"'-NHR1, -R"'-Epoxy,
- R"'-NCO, -R"'-NHCONHR¹, -R"'-NHCONR¹R² oder
- R"'-NH-COOR³,
wobei
R¹ und R² gleich oder verschieden sind und einen C₁-C₂₀ Alkyl, C₃-C₂₀-Cycloalkyl-, Aryl, C₇-C₁₈-Arylalkylrest, Oligo-/Polyethylen- und/oder Propylenglykole darstellen und R³ eine der Bedeutungen von R¹ hat oder einen Polyester- oder einen Polyamidrest bedeutet, wie z.B. Acetat- oder Butyl- und/oder Benzoeester, R"' einen aromatischen und/oder araliphatischen Rest bedeutet,
Z = Y, -Polyester-, -Polyether- und/oder -Polyamid-,
bedeutet,
und die gegebenenfalls weitere Additive enthalten,
und die weitere(n) Materialschichten auf diese Klebstoffschicht aufgelegt und die Klebstoffschicht(en) ausgehärtet wird/werden.

8. Verwendung von polymeren Systemen, die ausgewählt sind aus der Gruppe der Polyamide, Copolyamide, Polyolefine, Ethylenvinylacetat-Copolymere, Polyurethane, thermoplastische Polyurethane, Polyester, Polyacrylate und/oder Vinylpyrrolidon/Vinylacetat-Copolymere, Polyester-Urethane, Acrylate, Polyether und/oder Polyether- Urethane, und die zusätzlich ein Carbodiimid oder ein Gemisch aus mehreren Carbodiimide der Formeln (I), (II) und/oder (III) und gegebenenfalls weitere Additive enthalten, zum Verkleben von mindesten 2 unterschiedlichen Materialschichten zur Herstellung von flexible Verpackungen.

9. Verwendung nach Anspruch 8, **dadurch gekennzeichnet, dass** als Carbodiimid mindestens eine Verbindung der Formel (I) und/oder (II) eingesetzt wird, das erhältlich ist aus der Umsetzung von mindestens einem Carbodiimid der Formel (I) und/oder (II)), das über mindestens eine freie N-C-O-Funktionalität verfügt, mit mindestens einer aus nachwachsenden Rohstoffen isolierten oder daraus hergestellten H-aciden Verbindung und/oder einem Hydroxycarbonsäureester mit 2-24-Kohlenstoffatomen.

10. Verwendung der flexiblen Verpackung nach einem oder mehreren der Ansprüche 1 bis 6 für Lebensmittel, medizinische Anwendungen und/oder industrielle Produkte.
